# EUROPEAN PATENT SPECIFICATION

(11) **EP 2 758 368 B1**
(45) Date of publication and mention of the grant of the patent: **12.08.2015**
(21) Application number: 12729461.9
(22) Date of filing: 13.06.2012
(51) Int. Cl.: C07C 269/06, C07B 59/00, C07C 271/24

(54) **PREPARATION OF PET PRECURSOR**
HERSTELLUNG EINES PET-VORLÄUFERS
PRÉPARATION DE PRÉCURSEUR POUR PET

(30) Priority: 14.06.2011 US 201161496592 P
(43) Date of publication of application: 30.07.2014
(73) Proprietor: GE Healthcare Limited, Little Chalfont Buckinghamshire HP7 9NA (GB)
(72) Inventor: BERG, Tom, Christian, 0401 Oslo (NO)
(74) Representative: Bannan, Sally
(86) International application number: PCT/EP2012/061165
(87) International publication number: WO 2012/171941

(56) References cited:
- EP-A1- 1 978 015
- SHOUP T M ET AL: "Synthesis of ÄF-18Ü-1-amino-3-fluorocyclobutane-1-carbo xylic acid (FACBC): a PET tracer for tumor delineation", JOURNAL OF LABELLED COMPOUNDS AND RADIOPHARMACEUTICALS, JOHN WILEY, CHICHESTER, GB, vol. 42, no. 3, 1 January 1999 (1999-01-01), pages 215-225, XP003003890, ISSN: 0362-4803, DOI: 10.1002/(SICI)1099-1344(199903)42:3<215::A ID-JLCR180>3.0.CO;2-0

## Description

The invention relates to a process for preparation of radiopharmaceutical precursors, and in particular protected amino acid derivatives which are used as precursors for production of radio labelled amino acids for use in *in vivo* imaging procedures such as positron emission tomography (PET). Particularly, the invention relates to a process for preparation of a precursor of the [¹⁸F]-1-amino-3-fluorocyclobutanecarboxylic acid ([¹⁸F] FACBC) PET agent.

Nuclear medicine examination represented by positron emission tomography (PET) is effective in diagnosing a variety of diseases including heart diseases and cancer. These techniques involve administering an agent labeled with a specific radioisotope (hereinafter referred to as radiopharmaceutical) to a patient, followed by detecting γ-rays emitted directly or indirectly from the agent. Nuclear medicine examination is characteristic in that it has not only high specificity and sensitivity to diseases, but also an advantage of providing information on the functionality of lesions, compared to other examination techniques. For example, [¹⁸F]2-fluoro-2-deoxy-D-glucose ("[¹⁸F]FDG"), one radiopharmaceutical used for PET examination, tends to be concentrated in area where glucose metabolism is enhanced, thereby making it possible to specifically detect tumors in which glucose metabolism is enhanced. Nuclear medicine examination is performed by tracing a distribution of an administered radiopharmaceutical, and data obtained there from vary depending on nature of the radiopharmaceutical. Thus, different radiopharmaceuticals have been developed for different diseases, and some of them are put into clinical use. There have been developed, for example, various tumor diagnostic agents, bloodstream diagnostic agents and receptor mapping agents.

In recent years, a series of radioactive halogen-labeled amino acid compounds including [¹⁸F]-1-amino-3-fluorocyclobutanecarboxylic acid ([¹⁸F]FACBC) have been designed as novel radiopharmaceuticals. [¹⁸F]FACBC is considered to be effective as a diagnostic agent for highly proliferative tumors, because it has a property of being taken up specifically by amino acid transporters. Improved processes for preparation of [¹⁸F]FACBC and its precursors are sought.

EP1978015 (A1) provides processes for producing [¹⁸F] FACBC on a small scale. One of the intermediates in this process is 1-(N-(*t*-butoxycarbonyl) amino)-3-hydroxy-cyclobutane-1-carboxylic acid ethyl ester (Formula IV in Scheme 1 below). In the process step of EP1978015 (A1) for preparing this intermediate, dry palladium at neutral pH is used. Scheme 1 shows the multi-step synthesis, as outlined in EP1978015 (A1), for preparation of [¹⁸F] FACBC.

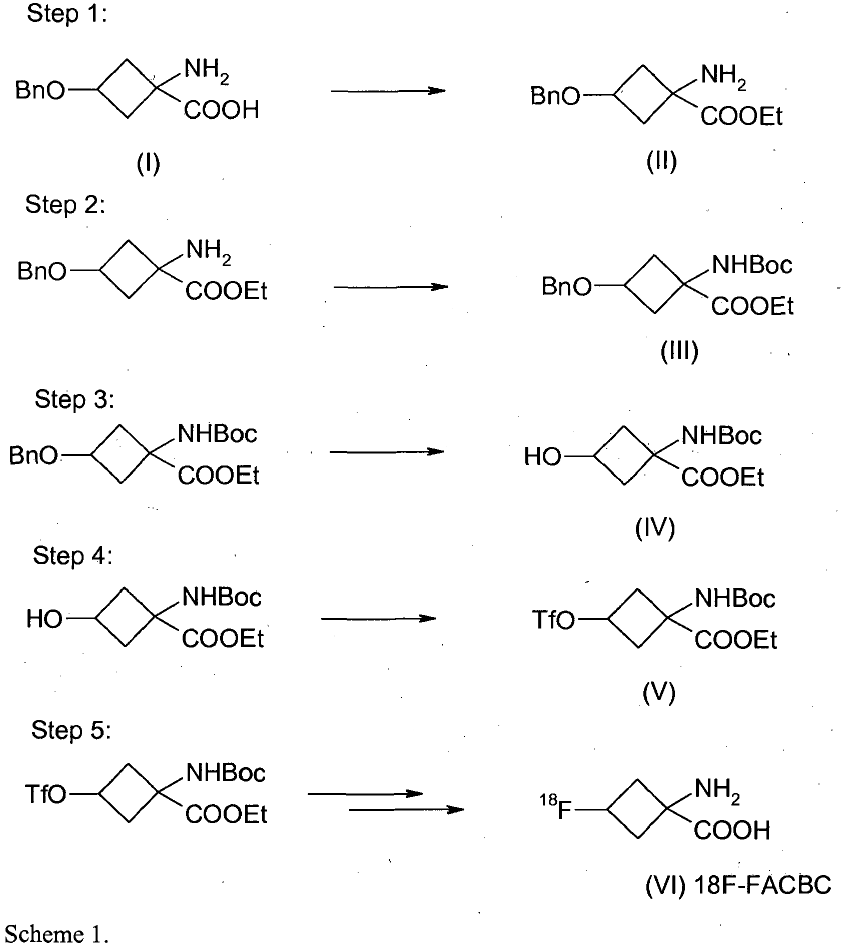

In Scheme 1 above, BnO denotes Benzyl ether, Boc denotes *tert*-butyl carbamate (tert-butoxycarbonyl) and OTf denotes Trifluoromethanesulfonate.

The last steps of the synthesis of [¹⁸F]FACBC, performed on an automated synthesiser unit, are based on nucleophilic displacement of a triflate group by [¹⁸F]fluoride from the precursor of Formula (V). The [¹⁸F]fluoride may be introduced with a solution of kryptofix (K222), potassium carbonate, water and acetonitrile into the reaction vessel. The ¹⁸F-labelled intermediate compound then undergoes two deprotecting steps, where the ethyl and the Boc protecting groups are removed by basic and acidic hydrolysis, respectively.

The compound of Formula (IV): 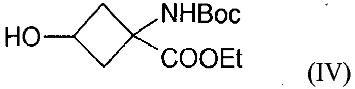 is named 1-(N-(*t*-butoxycarbonyl) amino)-3-hydroxy-cyclobutane-1-carboxylic acid ethyl ester. This intermediate is prepared by hydrogenolysis of 1-(N-(*t-*butoxycarbonyl) amino)-3-benzyloxy-cyclobutane-1-carboxylic acid ethyl ester (Formula III), as shown in step 3 of Scheme 1. Such hydrogenolysis, or debenzylation, may be performed by the use of a palladium catalyst and hydrogen gas. Such catalytic hydrogenation, using hydrogen gas as the hydrogenation source is well known, it is efficient, and as the [¹⁸F]FACBC compound and its precursors are prepared in a rather small scale, one should think there are no safety concerns using hydrogen gas for the hydrogenolysis step.

However, in the preparation of the compound of Formula (IV), it has been found that there are both process challenges and safety concerns that need to be dealt with, and in a hydrogenation facility specially-designed equipment and specially-designed rooms are needed. Hydrogen gas diffuses very easily, and it is challenging to ensure that the process equipment is tight, not leaking any gas. The equipment needs to withstand a certain overpressure, and hence specialised and expensive equipment is needed. Most importantly, hydrogen gas is highly reactive and needs to be carefully monitored in a controlled environment. Hence, hydrogenation detectors and alarms and special designed fire extinguishers are needed when using hydrogen gas.

Therefore, there is a need for a process for preparing the compound of Formula (IV) which is safe and which avoids the drawbacks associated with the use of hydrogen gas as the hydrogen source.

It has now surprisingly been found that the problem can be solved by using other sources of hydrogen than hydrogen gas in the hydrogenolysis step, and hence avoid the safety issues associated with using the gas, and at the same time achieve that the hydrogenolysis reaction goes to completion in an acceptable time period.

Therefore, in a first aspect the invention provides a process for preparation of a compound of Formula (IVa): 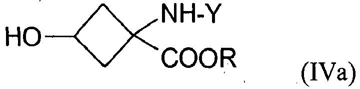 from a compound of Formula (IIIa) 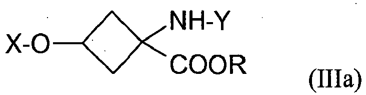 wherein
R denotes an alkyl group with 1 to 5 carbon atoms;
Y denotes a protecting group for an amine;
X denotes a protecting group for an alcohol;
wherein a reaction medium comprising the compound of Formula (IIIa) undergoes a catalytic hydrogenolysis of X of Formula (IIIa) using a non-gaseous hydrogen source.

The term "alkyl", alone or in combination, means a straight-chain or branched-chain alkyl radical having the general Formula CₙH₂ₙ₊₁. Examples of such radicals include methyl, ethyl, and isopropyl. The moiety R is preferably selected from methyl, ethyl, 1-propyl or isopropyl, and is most preferably ethyl.

The term "alcohol" herein refers to a substituent comprising the group -OH.

The term "amine" herein refers to the group NR'R" wherein R' and R" are independently hydrogen or an alkyl, and are preferably both hydrogen.

By the term "protecting group" is meant a group which inhibits or suppresses undesirable chemical reactions, but which is designed to be sufficiently reactive that it may be cleaved from the functional group in question to obtain the desired product under mild enough conditions that do not modify the rest of the molecule. Protecting groups are well known to those skilled in the art and are described in 'Protective Groups in Organic Synthesis', Theorodora W. Greene and Peter G. M. Wuts, (Fourth Edition, John Wiley & Sons, 2007).

A preferred amino protecting group for use in the present invention is selected from the group consisting of a t-butoxycarbonyl group, an allyloxycarbonyl group, a phthalimide group and N-benzylideneamine substituent. The Y moiety is hence a protecting group for an amine, such as for a carbamate.

The X moiety is a protecting group for alcohol, the protecting group is chosen so that the protecting group forms its related ether, such as; benzyl (Bn), benzyl carbonates, methoxymethyl (MOM), 2-methoxyethoxymethyl (MEM), methylthiomethyl (MTM), tetrahydropyranyl (THP), benzyloxymethyl (BOM), *p*-Methoxyphenyl, p-methoxybenzyl (MPM), *p*-methoxybenzyloxymethyl (PMBM), triisopropylsilyl (TIPS), *tert*-butyldimethylsilyl (TBDMS), 2-(trimethylsilyl)ethoxymethyl (SEM) and (phenyldimethylsilyl)methoxymethyl (SMOM). A group that can be removed by hydrogenation is preferred and in a preferred embodiment X is benzyl.

In a particularly preferred embodiment R is an ethyl group, Y is BOC and X is benzyl such that the compound of Formula IVa is a compound of Formula IV and the compound of Formula IIIa is a compound of Formula III, according to Scheme 1.

The "non-gaseous hydrogen source" is a compound that creates hydrogen gas when in contact with a catalyst, such as palladium on carbon. It is non-gaseous at ambient temperature when not in contact with a suitable catalyst. The hydrogen source is preferably selected from the group of cyclohexene, 1,4-dicyclohexadiene, ammonium formate (HCOONH₄), formic acid, triethylsilane (Et₃SiH) or a mixture of triethylsilane and triethylamine (Et₃SiH/Et₃N). More preferably the hydrogen source is cyclohexene, 1,4-dicyclohexadiene or triethylsilane and most preferably 1,4-dicyclohexadiene or triethylsilane. When using a mixture of Et₃SiH/Et₃N as the hydrogen source, the ratio between these should be 10:0-10:3, preferably 10:1-10:2 (Et₃SiH: Et₃N).

The "catalyst" used in the process of the invention is selected from the group of platinum metal group, and is accordingly selected from the group of ruthenium, rhodium, palladium, osmium, iridium, and platinum. More preferably, the catalyst is palladium.

The catalyst used in the process of the invention could be moistened to avoid any risk of ignition. If so, the catalyst used is preferably in the form of a moistened powder, and such moistened powder includes water. In one embodiment the moistened catalyst includes 30-70 % weight% water, more preferably 40-60 weight% water, and most preferably 45-55 weight% water. In a particularly preferable embodiment the wet catalyst includes about 50 weight% water.

Further, the catalyst used is preferably a heterogeneous catalyst, meaning that it includes solid particles of the metal which is suspended in the reaction medium. The catalyst used in the invention, such as palladium, is preferably distributed over finely divided carbon, referred to as palladium on carbon (Pd/C), Pd(OH)₂ on carbon (Pearlman's catalyst) or Pd(OAc)₂. Such catalysts are commercially available with a metal loading of 1 - 30 %, and these can be used in the method of the invention. The metal loading, such as the palladium loading, is more preferably 1-10% and most preferably 5-10 %. The amount of catalyst to be used in the process depends on which catalyst is chosen, and on the percentage of loading. With e.g. a 10 % loaded palladium on carbon catalyst, the amount of catalyst to be used in the method of the invention is 1-30 weight%/compound, more preferably 5-20 weight%/compound and most preferably around 10 weight%/compound. The "compound" referred to here is the start material, i.e. a compound of Formula IIIa, such as the compound of Formula III.

When using the process of the invention, it has surprisingly been found that the hydrogenolysis reaction (step 3) goes to completion in an acceptably short time, at the same time as the protecting groups of the amine function (group Y) and the ester group (group R)were not affected. The Y protecting group is later to be removed by acidic hydrolysis, and it is crucial that it is not removed during the hydrogenolysis step of the process of the invention.

In the process of the invention the compound of Formula IIIa, such as the compound of Formula III, is dissolved in a solvent. Such solvent is a polar solvent, either protic or aprotic, and is preferably selected from the group of alcohols, esters, ethers and chlorinated solvents, and it is more preferably an alcohol and most preferably ethanol. The amount of solvent should be sufficient to completely dissolve the compound of Formula IIIa. The mol/ml ratio between the compound of Formula IIIa and the solvent is e.g. between 1:4 and 1:8.

In one embodiment, an acid is further added to the solution comprising the compound of Formula IIIa to adjust the pH, preferably a mineral acid or an organic acid. The acid is preferably selected from the group of hydrochloric acid, acetic acid, formic acid and sulphuric acid. Most preferably the acid is acetic acid. The pH is preferably measured and adjusted to the wanted level by the addition of this acid to the reaction medium. In one embodiment, the pH of the reaction medium is adjusted to 2.0-5.0, more preferably 2.5-3.5 and most preferably to about 3.

The hydrogen source is added to the reaction medium comprising the compound of Formula IIIa, the solvent, the optional acid and the catalyst. The reaction medium, including the hydrogen source, should be effectively stirred during the reaction. The amount of hydrogen source needed depends on which hydrogen source is to be used. In one embodiment the mol ratio between the hydrogen source and the compound of Formula IIIa, is 20:1 - 5:1, such as e.g. about 10:1.In one embodiment, the reaction medium is gently heated to ensure the reaction proceeds at a convenient rate. The need for heating depends on which hydrogen source is used. 1,4-cyclohexadiene, formic acid and Et₃SiH/Et₃N are expected to work efficiently at ambient temperature, while the use of cyclohexene or ammonium formate in the hydrogenolysis reaction may require some heating. In one embodiment, the reaction medium is e.g. heated to above room temperature, such as up to 80°C, more preferably up to about 60°C. Such heating may be performed by reflux. Hence, the compound of Formula (IIIa), such as the compound of Formula (III), is dissolved in a solvent as described above, e.g. an alcohol, mixed with a hydrogen source, e.g. cyclohexene, and added the catalyst, e.g. Pd(OH)₂, and this reaction mixture is then refluxed. The catalyst could then be filtered off, washed and the filtrate is then evaporated, removing solvents and remains from the hydrogen donor.

The process of the invention can be used in all scales and is particularly useful when preparing in large scale, such as when preparing 100 grams or more, such as 300 grams, or up to 500 grams or more, of the compound of Formula (IVa). In smaller scales, it is well known to use hydrogen gas in step 3 of Scheme 1, however when scaling up, from a safety perspective it is advantageous to use the claimed invention. The process of the invention including non-gaseous sources of hydrogen is much safer, and likely more efficient and cost efficient as there are lower equipment requirements. When no externally applied gas is used, there is no need for an overpressure in the process equipment, and no need for special equipment withstanding such overpressure. Further, when using the process of the invention there are no negative consequences concerning purification, as the reaction product from the hydrogen source can easily be removed by evaporation, e.g. together with evaporation of the solvent used in the reaction.

When performing the process of the invention the hydrogenolysis goes to completion, such as in five days or less, preferably in four days or less and most preferably in 3 days or less.

In a further aspect, the invention provides a process for preparing the precursor compound of ¹⁸F-FACBC, according to Formula (V): 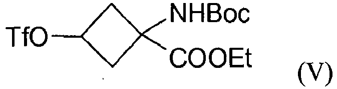 including a step of preparing the compound of Formula (IVa) according to the process of the first aspect. OTf denotes trifluoromethanesulfonate. Y in Formula IVa is then Boc and R is ethyl.

The invention is illustrated by way of the following examples:
Example 1: 1-(N-(*t*-butoxycarbonyl) amino)-3-benzyloxy-cyclobutane-1-carboxylic acid ethyl ester (Compound of Formula III) is dissolved in ethanol (4 ml/ 1.0 mmol substrate, abs) and applied a gentle N₂-atmosphere. The catalyst Pd/C (10%) is added to the reaction mixture followed by addition of 1,4-cyclohexadiene (10 mmol/ 1 mmol substrate), the resulting mixture is stirred at ambient temperature, and the reaction is monitored by in-process TLC analysis. Upon complete reaction the mixture is filtered through Celite, washed with ethanol and evaporated *in vacuo* to dryness.
Example 2: 1-(N-(*t*-butoxycarbonyl) amino)-3-benzyloxy-cyclobutane-1-carboxylic acid ethyl ester (Compound of Formula III) in methanol is added Pd/C (10%). The resulting mixture is added Et₃SiH (10 mmol/l mmol substrate) dropwise under an argon atmosphere. The reaction progress is monitored by TLC analysis, and upon complete reaction the mixture is filtered through Celite, washed with methanol and evaporated *in vacuo* to dryness.

## Claims

1. A process for preparation of a compound of Formula (IVa): 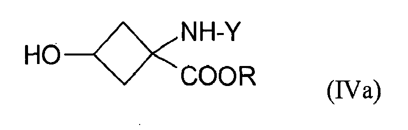 from a compound of Formula (IIIa) 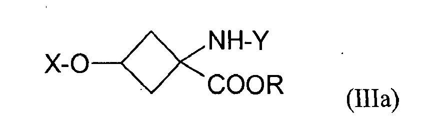 wherein:
R denotes an alkyl group with 1 to 5 carbon atoms;
Y denotes a protecting group for an amine;
X denotes a protecting group for an alcohol;
wherein a reaction medium comprising the compound of Formula (IIIa) undergoes a catalytic hydrogenolysis of X of Formula (IIIa) using a non-gaseous hydrogen source.

2. A process as claimed in claim 1 wherein R is an ethyl group, Y is BOC and X is benzyl.

3. A process as claimed in any of claims 1 or 2 wherein the reaction medium further comprises the hydrogen source and the catalyst.

4. A process as claimed in any of claims 1 to 3 wherein the non-gaseous hydrogen source is selected from the group cyclohexene, 1,4-dicyclohexadiene, ammonium formate (HCOONH₄), formic acid, triethylsilane (Et₃SiH) or a mixture of triethylsilane and triethylamine (Et₃SiH/Et₃N).

5. Process as claimed in any of claims 1 to 4 wherein the non-gaseous hydrogen source is 1,4-dicyclohexadiene or triethylsilane.

6. A process as claimed in any of claims 1 to 5 wherein the catalyst is selected from the group of ruthenium, rhodium, palladium, osmium, iridium, and platinum.

7. A process as claimed in any of claims 1 to 6 wherein the catalyst is palladium.

8. A process as claimed in any of claims 1 to 7 wherein the catalyst is palladium on carbon with a palladium loading of 1-10 %.

9. A process as claimed in any of claims 1 to 8 wherein the reaction medium further comprises a solvent selected from the group of alcohols, esters, ethers and chlorinated solvents.

10. A process as claimed in claim 9 wherein the solvent is ethanol.

11. A process as claimed in any of claims 1 to 10 wherein the reaction mixture is effectively stirred during the hydrogenolysis.

12. A process as claimed in any of claims 1 to 11 wherein the reaction product of the hydrogen source is evaporated off after the process has completed.

13. A process for preparing the precursor compound of Formula (V) of ¹⁸F-FACBC: 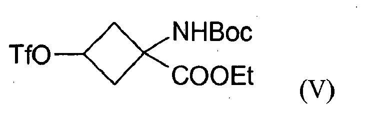 wherein TfO denotes trifluoromethanesulfonate,
including a step of preparing the compound of Formula (IVa) as claimed in any of the claims 1 to 12.

## Patentansprüche

1. Verfahren zur Herstellung einer Verbindung der Formel (IVa): 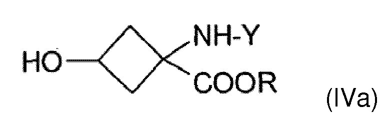 ausgehend von einer Verbindung der Formel (IIIa) 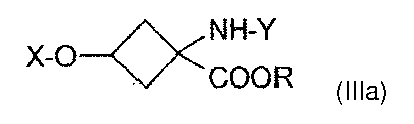 wobei:
R für eine Alkylgruppe mit 1 bis 5 Kohlenstoffatomen steht;
Y für eine Schutzgruppe für ein Amin steht;
X für eine Schutzgruppe für einen Alkohol steht;
wobei ein Reaktionsmedium umfassend die Verbindung der Formel (IIIa) unter Verwendung einer nicht gasförmigen Wasserstoffquelle eine katalytische Hydrogenolyse von X in Formel (IIIa) durchläuft.

2. Verfahren nach Anspruch 1, wobei R eine Ethylgruppe, ist, Y BOC ist und X Benzyl ist.

3. Verfahren nach einem der Ansprüche 1 oder 2, wobei das Reaktionsmedium weiterhin die Wasserstoffquelle und den Katalysator umfasst.

4. Verfahren nach einem der Ansprüche 1 bis 3, wobei die nicht gasförmige Wasserstoffquelle ausgewählt ist aus der Gruppe bestehend aus Cyclohexen, 1,4-Dicyclohexadien, Ammoniumformiat (HCOONH₄), Ameisensäure, Triethylsilan (Et₃SiH) oder einer Mischung aus Triethylsilan und Triethylamin (Et₃SiH/Et₃N).

5. Verfahren nach einem der Ansprüche 1 bis 4, wobei die nicht gasförmige Wasserstoffquelle 1,4-Dicyclohexadien oder Triethylsilan ist.

6. Verfahren nach einem der Ansprüche 1 bis 5, wobei der Katalysator ausgewählt ist aus der Gruppe bestehend aus Ruthenium, Rhodium, Palladium Osmium, Iridium und Platin.

7. Verfahren nach einem der Ansprüche 1 bis 6, wobei der Katalysator Palladium ist.

8. Verfahren nach einem der Ansprüche 1 bis 7, wobei der Katalysator Palladium auf Kohlenstoff mit einer Palladiumbeladung von 1 bis 10 % ist.

9. Verfahren nach einem der Ansprüche 1 bis 8, wobei das Reaktionsmedium weiterhin ein Lösungsmittel ausgewählt aus der Gruppe bestehend aus Alkoholen, Estern, Ethern und chlorierten Lösungsmitteln enthält.

10. Verfahren nach Anspruch 9, wobei das Lösungsmittel Ethanol ist.

11. Verfahren nach einem der Ansprüche 1 bis 10, wobei die Reaktionsmischung während der Hydrogenolyse effektiv gerührt wird.

12. Verfahren nach einem der Ansprüche 1 bis 11, wobei das Reaktionsprodukt der Wasserstoffquelle verdampft wird nachdem das Verfahren beendet ist.

13. Verfahren zum Herstellen der Vorläuferverbindung der Formel (V) von ¹⁸F-FACBC: 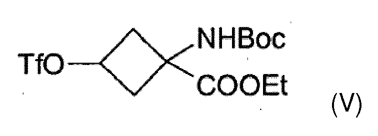 wobei TfO für Trifluormethansulfonat steht,
einschließlich eines Schritts des Herstellens der Verbindung der Formel (IVa) nach einem der Ansprüche 1 bis 12.

## Revendications

1. Procédé de préparation d'un composé de formule
(IVa) : 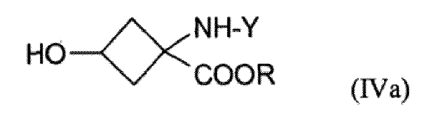 À partir d'un composé de formule (IIIa) : 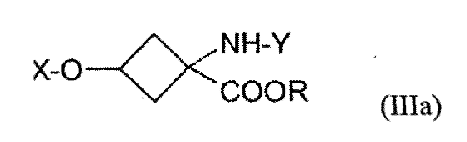 dans lequel :
R désigne un groupement alkyle avec 1 à 5 atomes de carbone ;
Y désigne un groupement protecteur pour une amine ;
X désigne un groupement protecteur pour un alcool ;
dans lequel un milieu réactionnel comprenant le composé de formule (IIIa) subit une hydrogénolyse catalytique de X de formule (IIIa) en utilisant une source d'hydrogène non gazeuse.

2. Procédé selon la revendication 1, dans lequel R est un groupement éthyle, Y est BOC et X est un groupement benzyle.

3. Procédé selon l'une quelconque des revendications 1 ou 2, dans lequel le milieu réactionnel comprend en outre la source d'hydrogène et le catalyseur.

4. Procédé selon l'une quelconque des revendications 1 à 3, dans lequel la source d'hydrogène non gazeuse est choisie dans les groupements suivants : cyclohexène, 1,4-dicyclohexadiène, formiate d'ammonium (HCOONH₄), acide formique, triéthylsilane (Et₃SiH) ou un mélange de triéthylsilane et de triéthylamine (Et₃SiH :Et₃N).

5. Procédé selon l'une quelconque des revendications 1 à 4, dans lequel la source d'hydrogène non gazeuse est le 1,4-dicyclohexadiène ou le triéthylsilane.

6. Procédé selon l'une quelconque des revendications 1 à 5, dans lequel le catalyseur est choisi dans le groupe du ruthénium, du rhodium, du palladium, de l'osmium, de l'iridium et du platine.

7. Procédé selon l'une quelconque des revendications 1 à 6, dans lequel le catalyseur est le palladium.

8. Procédé selon l'une quelconque des revendications 1 à 7, dans lequel le catalyseur est le palladium sur du carbone avec une charge de palladium de 1 à 10 %.

9. Procédé selon l'une quelconque des revendications 1 à 8, dans lequel le milieu réactionnel comprend en outre un solvant choisi dans le groupe des alcools, des esters, des éthers et des solvants chlorés.

10. Procédé selon la revendication 9, dans lequel le solvant est l'éthanol.

11. Procédé selon l'une quelconque des revendications 1 à 10, dans lequel le mélange réactionnel est efficacement agité au cours de l'hydrogénolyse.

12. Procédé selon l'une quelconque des revendications 1 à 11, dans lequel le produit réactionnel de la source d'hydrogène est évaporé une fois le procédé terminé.

13. Procédé de préparation du composé précurseur de formule (V) du ¹⁸F-FACBC : 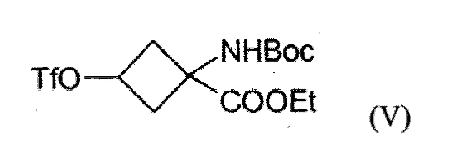 dans lequel TfO désigne le trifluorométhanesulfonate,
comprenant une étape de préparation du composé de formule (IVa) selon l'une quelconque des revendications 1 à 12.
